# EUROPEAN PATENT APPLICATION

(11) **EP 1 188 447 A1**
(43) Date of publication of application: **20.03.2002**
(21) Application number: 00937272.3
(22) Date of filing: 16.06.2000
(51) Int. Cl.: A61K 45/00, A61K 31/421, A61K 31/422, A61K 31/27, A61K 31/428, A61P 17/02

(54) **REMEDIES FOR SKIN ULCER**

(30) Priority: 21.06.1999 JP 17376399
(71) Applicant: FUJISAWA PHARMACEUTICAL CO., LTD., Osaka-shi Osaka 541-8514 (JP)
(72) Inventor: HIGAKI, Masahide, Hirakata-shi, Osaka 573-0171 (JP); IMAMURA, Emiko, Nishinomiya-shi, Hyogo 663-8103 (JP)
(74) Representative: Gille Hrabal Struck Neidlein Prop Roos
(86) International application number: JP0003935
(87) International publication number: WO0078350

(57) **Abstract**

The invention provides an agent for the prevention and/or treatment of skin ulcer or bedsore which comprises a nonprostanoid prostaglandin I₂ agonist as an active ingredient.

## Description

### TECHNICAL FIELD

The present invention relates to a pharmaceutical composition comprising a nonprostanoid prostaglandin I₂ agonist as an active ingredient.

The present invention relates to a novel use of a nonprostanoid prostaglandin I₂ agonist.

More particularly, it is an object of the present invention to provide a pharmaceutical composition which comprises a nonprostanoid prostaglandin I₂ agonist as an active ingredient and is intended for use in the prevention and/or treatment of skin ulcer [e.g. diabetic skin ulcer, inclusive of ulcer of lower limb, burn ulcer (burn), traumatic ulcer, crural (cnemial) ulcer, diabetic gangrene, etc.] and decubitus ulcer (bedsore) and the like, particularly diabetic skin ulcer, in humans or animals.

### DISCLOSURE OF INVENTION

The inventors of this invention found that nonprostanoid prostaglandin I₂ agonists are useful in the prevention and/or treatment of skin ulcer [e.g. diabetic skin ulcer, inclusive of ulcer of lower limb, burn ulcer (burn), traumatic ulcer, crural (cnemial) ulcer, diabetic gangrene, etc.] and decubitus ulcer (bedsore), particularly diabetic skin ulcer, in humans or animals and, based on this finding, they have now completed the present invention.

The present invention is practiced by preparing a pharmaceutical composition comprising a nonprostanoid prostaglandin I₂ agonist as an active ingredient and administering the same to humans or animals for the prevention and/or treatment of skin ulcer [e.g. diabetic skin ulcer, inclusive of ulcer of lower limb, burn ulcer (burn), traumatic ulcer, crural (cnemial) ulcer, diabetic gangrene, etc.] and decubitus ulcer (bedsore), particularly diabetic skin ulcer.

The nonprostanoid prostaglandin I₂ agonist to be used in accordance with this invention is a prostaglandin I₂ agonist having no prostaglandin skeleton or bicylco[3.3.0]octane skeleton or 2-oxabicyclo[3.3.0]octane skeleton.

Preferred as the nonprostanoid prostaglandin I₂ agonist in the practice of the invention are the following compounds (I): 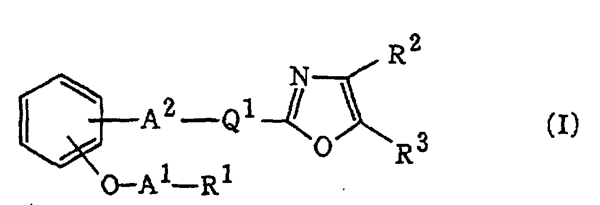 [wherein
R¹ is carboxy or protected carboxy,
R² is aryl which may optionally have one or more suitable substituents,
R³ is aryl which may optionally have one or more suitable substituents,
A¹ is lower alkylene,
A² is a single bond or lower alkylene and
-Q¹- is 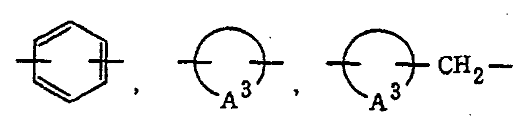
or 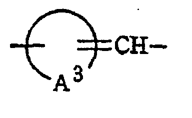 (in which 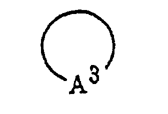 represents cyclo(lower)alkane or cyclo(lower)alkene, which respectively may optionally have one or more suitable substituents)]; or the following compounds (II): 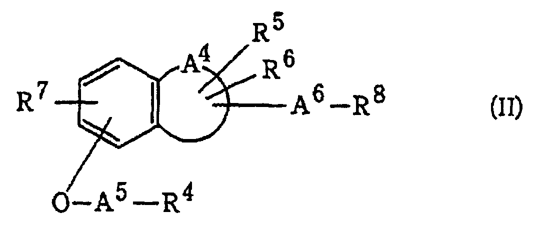 [wherein
R⁴ is carboxy or protected carboxy,
R⁵ is hydrogen, hydroxy or protected hydroxy,
R⁶ is hydrogen, hydroxy, protected hydroxy, lower alkyl or halogen,
R⁷ is hydrogen or halogen,
A⁵ is lower alkylene,
A⁶ is a single bond or lower alkylene and
-R⁸ is 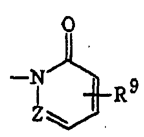
(in which R⁹ is mono(or di or tri)aryl(lower)alkyl and Z is N or CH) or 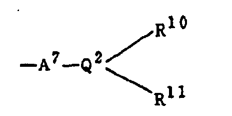 (in which A⁷ is 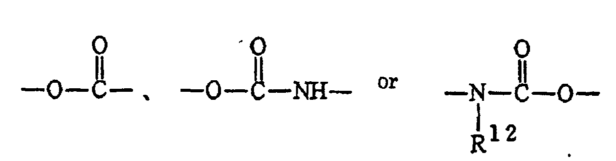 (in which R¹² is hydrogen or lower alkyl), Q² is N or CH, R¹⁰ is aryl and R¹¹ is aryl), and 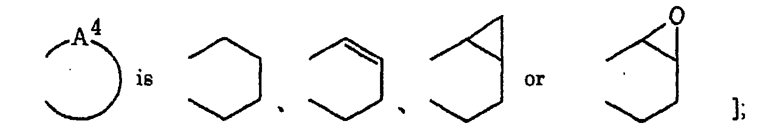 or the following compounds (III): 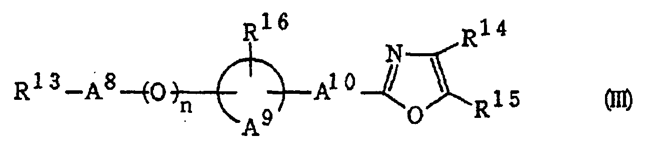 [wherein
R¹³ is carboxy or protected carboxy,
R¹⁴ is aryl which may optionally have one or more suitable substituents,
R¹⁵ is aryl which may optionally have one or more suitable substituents,
R¹⁶ is hydrogen, lower alkyl, hydroxy or aryl,
A⁸ is lower alkylene, 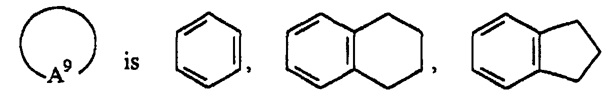 or 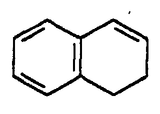
-A¹⁰- is 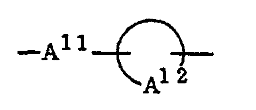
(in which -A¹¹- is a single bond, -CH₂- or -CO-, 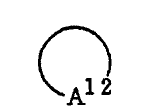 represents cyclo(C5-C8)alkene, cyclo(C7-C8)alkane, bicycloheptane, bicycloheptene, tetrahydrofuran, tetrahydrothiophene, azetidine, pyrrolidine or piperidine, which respectively may optionally have one or more suitable substituents) or -X-A¹³- (in which -X- is -O-, -S-, or -N(R¹⁷)- (R¹⁷ being hydrogen, lower alkyl or acyl) and A¹³ is lower alkylene which may optionally have one or more suitable substituents) and n is 0 or 1];
or pharmaceutically acceptable salts thereof.

Suitable salts for use as the pharmaceutically acceptable salts of compounds (I) to (III) are conventional nontoxic salts, including alkali metal salts (e.g. sodium salt, potassium salt, etc.), alkaline earth metal salts (e.g. calcium salt, magnesium salt, etc.), ammonium salt, organic base salts (e.g. trimethylamine salt, triethylamine salt, pyridine salt, picoline salt, dicyclohexylamine salt, N,N'-dibenzylethylenediamine salt, etc.), organic acid salts (e.g. acetate, maleate, tartrate, methanesulfonate, benzenesulfonate, formate, toluenesulfonate, trifluoroacetate, etc.), inorganic acid salts (e.g. hydrochloride, hydrobromide, sulfate, phosphate, etc.), salts with amino acids (e.g. arginine, aspartic acid, glutamic acid, etc.) and the like.

The compounds (I) to (III) and pharmaceutically acceptable salts thereof, which are to be used in accordance with this invention, may contain one or more asymmetric centers and therefore they may exist as enantiomers or diastereoisomers. Both mixtures of such isomers and individual isomers fall within the scope of this invention.

The compounds (I) to (III) and pharmaceutically acceptable salts thereof, which are to be used in accordance with this invention, may exist a solvate and this solvate also falls within the scope of this invention. As preferred solvate, there may be mentioned hydrates, ethanolates and the like.

Radiolabeled derivatives of the compounds (I) to (III), which are suited for use in biological studies, also fall within the scope of this invention.

Referring to the above description and subsequent description in the present specification, suitable examples and specific examples falling within respective definitions given herein for defining the scope of the present invention are shown below in detail.

Unless otherwise specified, the term "lower" means that 1 to 6 carbon atoms are involved.

Suitable species of "aryl" and of "aryl moiety" of "mono(or di or tri)aryl(lower)alkyl" are phenyl, naphthyl and the like.

Preferred as "lower alkylene" are straight or branched ones containing 1 to 6 carbon atoms, such as methylene, ethylene, trimethylene, tetramethylene, pentamethylene and hexamethylene. More preferred are those containing 1 to 3 carbon atoms.

Preferred as "lower alkyl" or as "lower alkyl moiety" of "mono(or di or tri)aryl(lower)alkyl" are straight or branched ones containing 1 to 6 carbon atoms, such as methyl, ethyl, propyl, isopropyl, butyl, isobutyl, sec-butyl, tert-butyl, pentyl, tert-pentyl and hexyl. More preferred are those containing 1 to 4 carbon atoms.

Preferred as "protected carboxy" are esterified carboxy etc.

Preferred examples of the ester moiety of the esterified carboxy are (1) lower alkyl esters (e.g. methyl ester, ethyl ester, propyl ester, isopropyl ester, butyl ester, isobutyl ester, tert-butyl ester, pentyl ester, hexyl ester, etc.), which may optionally have at least one suitable substituent, thus including, for example, lower alkanoyloxy(lower)alkyl esters [e.g. acetoxymethyl ester, propionyloxymethyl ester, butyryloxymethyl ester, valeryloxymethyl ester, pivaloyloxymethyl ester, hexanoyloxymethyl ester, 1-(or 2-)acetoxyethyl ester, 1-(or 2- or 3-)acetoxypropyl ester, 1-(2-, 3- or 4-)acetoxybutyl ester, 1-(or 2-)propionyloxyethyl ester, 1-(or 2- or 3-)propionyloxypropyl ester, 1-(or 2-)butyryloxyethyl ester, 1-(or 2-)isobutyryloxyethyl ester, 1-(or 2-)pivaloyloxyethyl ester, 1-(or 2-)hexanoyloxyethyl ester, isobutyryloxymethyl ester, 2-ethylbutyryloxymethyl ester, 3,3-dimethylbutyryloxymethyl ester, 1-(or 2-)pentanoyloxyethyl ester, etc.], lower alkylsulfonyloxy(lower)alkyl esters (e.g. 2-mesylethyl ester etc.), mono(or di- or tri-)halo(lower)alkyl esters (e.g. 2-iodoethyl ester, 2,2,2-trichloroethyl ester, etc.), lower alkoxycarbonyloxy(lower)alkyl esters (e.g. methoxycarbonyloxymethyl ester, ethoxycarbonyloxymethyl ester, 2-methoxycarbonyloxyethyl ester, 1-ethoxycarbonyloxyethyl ester, 1-isopropoxycarbonyloxyethyl ester, etc.), phthalidylidene(lower)alkyl esters, (5-(lower)alkyl-2-oxo-1,3-dioxol-4-yl)(lower)alkyl esters [e.g. (5-methyl-2-oxo-1,3-dioxol-4-yl)methyl ester, (5-ethyl-2-oxo-1,3-dioxol-4-yl)methyl ester, (5-propyl-2-oxo-1,3-dioxol-4-yl)ethyl ester, etc.];
(2) lower alkenyl esters (e.g. vinyl ester, allyl ester, etc.);
(3) lower alkynyl esters (e.g. ethynyl ester, propynyl ester, etc.);
(4) ar(lower)alkyl esters, which may optionally have at least one suitable substituent, for example mono(or di- or tri-)phenyl(lower)alkyl esters, which may optionally have at least one suitable substituent, (e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-tert-butylbenzyl ester, etc.);
(5) aryl esters which may optionally have at least one suitable substituent (e.g. phenyl ester, 4-chlorophenyl ester, tolyl ester, tert-butylphenyl ester, xylyl ester, mesityl ester, cumenyl ester, etc.);
(6) phthalidyl ester, and so forth.

Referring to the expression "aryl which may optionally have at least one suitable substituent", halogen, amino, hydroxy, lower alkoxy and such lower alkyl species as mentioned above are preferred as "substituent".

Suitable examples of "cyclo(lower)alkane" are cyclopropane, cyclobutane, cyclopentane and cyclohexane.

Suitable examples of "cyclo(lower)alkene" are cyclopropene, cyclobutene, cyclopentene and cyclohexene.

Referring to the expression "cyclo(lower)alkane or cyclo(lower)alkene, which respectively may optionally have one or more suitable substituents", epoxy, hydroxy, lower alkoxy and the like are preferred as "substituents".

Suitable examples of "lower alkoxy" are methoxy, ethoxy, propoxy, isopropoxy, butoxy, isobutoxy, tert-butoxy, pentyloxy, tert-pentyloxy, hexyloxy and the like.

Preferred as "protected hydroxy" are acyloxy etc.

Preferred as "acyl" and as "acyl moiety" of "acyloxy" are aliphatic acyl groups and acyl groups having an aromatic or heterocyclic ring.

As preferred examples of the acyl, there may be mentioned lower alkanoyl groups (e.g. formyl, acetyl, propionyl, butyryl, isobutyryl, valeryl, isovaleryl, oxalyl, succinyl, pivaloyl, etc.);
lower alkoxycarbonyl groups (e.g. methoxycarbonyl, ethoxycarbonyl, propoxycarbonyl, isopropoxycarbonyl, butoxycarbonyl, tert-butoxycarboyl, pentyloxycarbonyl, hexyloxycarbonyl, etc.):
lower alkylsulfonyl groups (e.g. mesyl, ethanesulfony, propanesulfonyl, ispropanesulfonyl, butanesuflonyl, etc.);
arenesulfonyl groups (e.g. benzenesulfonyl, tosyl, etc.);
aroyl groups (e.g. benzoyl, toluoyl, xyloyl, naphthoyl, phthaloyl, indanecarbonyl, etc.);
ar(lower)alkanoyl groups (e.g. phenylacetyl, phenylpropionyl, etc.); ar(lower)alkoxycarbonyl groups (e.g. benzyloxycarbonyl, phenethyloxycarbonyl, etc.); and so on.

Suitable examples of "halogen" are chlorine, bromine, iodine and fluorine.

Preferred species of "cyclo(C5-C8)alkene" are cyclopentene, cyclohexene, cyclopentene and cyclooctene.

Preferred species of "cyclo(C7-C8)alkane" are cycloheptane and cyclooctane.

Preferred as "bicyclcoheptane" are bicyclo[2.2.1]heptane etc.

Preferred as "bicycloheptene" are bicyclo[2.2.1]heptene such as bicyclo[2.2.1]hept-2-ene, and the like.

Referring to the expression "cyclo(C5-C8)alkene, cyclo(C7-C8)alkane, bicycloheptane, bicycloheptene, tetrahydrofuran, tetrahydrothiophene, azetidine, pyrrolidine or piperidine, which respectively may optionally have one or more suitable substituents", imino, hydroxy, oxo, such acyl species as mentioned above, imino-protecting groups and the like are preferred as "substituents".

Suitable "imino-protecting groups" are mono(or di or tri)aryl(lower)alkyl groups and the like.

Referring to the expression "lower alkylene which may optionally have one or more suitable substituents", suitable "substituents" include such lower alkyl species as mentioned above, hydroxy lower alkyl (e.g. hydroxymethyl, hydroxyethyl, hydroxypropyl, hydroxybutyl, hydroxypentyl, hydroxyhexyl, etc.) and the like.

Preferred embodiments of compounds (I) are as follows:
Those in which R¹ is carboxy, or protected carboxy (more preferably esterified carboxy, most preferably lower alkoxycarbonyl),
R² is aryl, which may optionally have one to three (more preferably one) suitable substituents (more preferably phenyl or lower alkylphenyl),
R³ is aryl which may optionally have one to three (more preferably one) suitable substituents (more preferably phenyl or lower alkylphenyl),
A¹ is lower alkylene (more preferably C1-C3 alkylene, most preferably methylene),
A² is a single bond, or lower alkylene (more preferably C1-C3 alkylene, most preferably methylene), and
Q¹- is 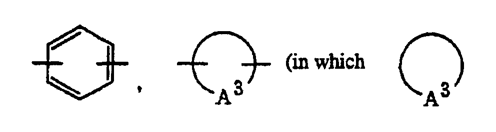
is cyclo(lower)alkane which may optionally have one substituent selected from the group consisting of epoxy, hydroxy and lower alkoxy, or cyclo(lower)alkene) or 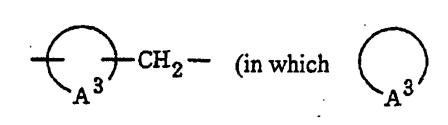 is cyclo(lower)alkane which may optionally have one substituent selected from the group consisting of epoxy and hydroxy, or cyclo(lower)alkene) or 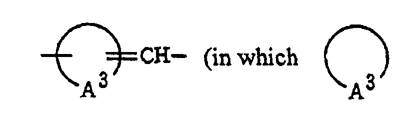 is cyclo(lower)alkane.

Preferred embodiments of compounds (II) are as follows:
Those in which R⁴ is carboxy, or protected carboxy (more preferably esterified carboxy, most preferably lower alkoxycarbonyl),
R⁵ is hydrogen, hydroxy, or protected hydroxy (more preferably acyloxy),
R⁶ is hydrogen, hydroxy, protected hydroxy (more preferably acyloxy), lower alkyl or halogen,
A⁵ is lower alkylene (more preferably C1-C3 alkylene, most preferably methylene),
A⁶ is a single bond, or lower alkylene (more preferably C1-C3 alkylene, most preferably methylene or ethylene),
-R⁸ is 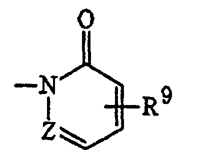
(in which R⁹ is diaryl(lower)alkyl (more preferably diphenyl(lower)alkyl, most preferably diphenylmethyl) and Z is N or CH) or 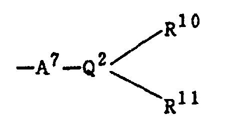 (in which -A⁷- is 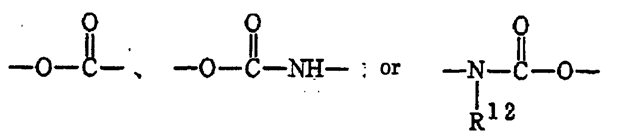
(R¹² being hydrogen or lower alkyl),
Q² is N or CH,
R¹⁰ is aryl (more preferably phenyl),
R¹¹ is aryl (more preferably phenyl) and

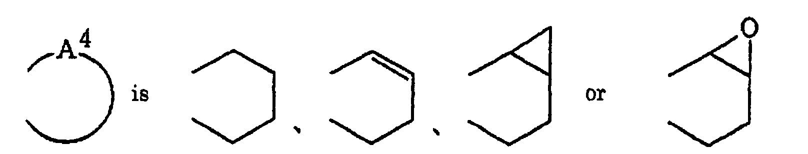

Preferred embodiments of compounds (III) are as follows:
Those in which R¹³ is carboxy, or protected carboxy (more preferably esterified carboxy, most preferably lower alkoxycarbonyl),
R¹⁴ is aryl which may optionally have lower alkyl (more preferably phenyl or lower alkylphenyl, most preferably phenyl or C1-C4 alkylphenyl),
R¹⁵ is aryl which may optionally have lower alkyl (more preferably phenyl or lower alkylphenyl, most preferably phenyl or C1-C4 alkylphenyl),
R¹⁶ is hydrogen, lower alkyl (more preferably C1-C4 alkyl, most preferably methyl), hydroxy or aryl (more preferably phenyl),
A⁸ is lower alkylene (more preferably C1-C4 alkylene, most preferably methylene or ethylene). 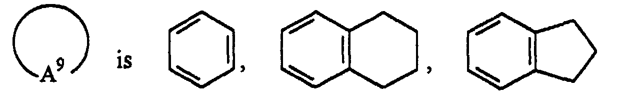 or 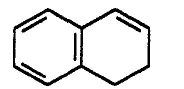
-A¹⁰- is
(in which -A¹¹- is a single bond, -CH₂- or -CO-, 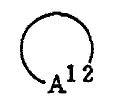 is cyclo(C5-C8)alkene, cyclo(C7-C8)alkane, bicycloheptane (more preferably bicyclo[2.2.1]heptane), bicycloheptene (more preferably bicyclo[2.2.1]heptene, most preferably bicyclo[2.2.1]hept-2-ene), tetrahydrofuran, tetrahydrothiophene, azetidine, pyrrolidine or piperidine, which respectively may optionally have one to three (more preferably one or two) substituents each selected from the group consisting of imino, oxo, acyl (more preferably lower alkanoyl, most preferably C1-C4 alkanoyl) and imino protecting groups (more preferably mono(di or tri)phenyl(lower)alkyl, most preferably phenyl(lower)alkyl) or -X-A¹³- (in which -X- is -O-, -S-, or -N(R¹⁷)- (R¹⁷ being hydrogen, lower alkyl(more preferably C1-C4 alkyl) or acyl (more preferably lower alkanoyl, most preferably C1-C4 alkanoyl)) and A¹³ is lower alkylene (more preferably C1-C4 alkylene, most preferably methylene or ethylene) which may optionally have one to three (more preferably one) suitable substituents selected from the group consisting of lower alkyl (more preferably C1-C4 alkyl) and hydroxy(lower)alkyl (more preferably hydroxy(C1-C4)alkyl)) and n is 0 or 1.

More preferred among compounds (III) are compounds of the following formula (III-A): 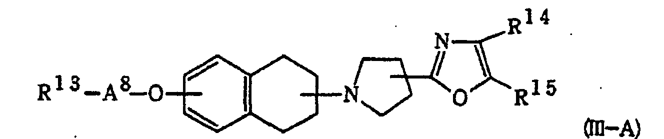 wherein R¹³ is carboxy, or protected carboxy (more preferably esterified carboxy, most preferably lower alkoxycarboyl),
R¹⁴ is phenyl, or lower alkylphenyl (more preferably C1-C4 alkylphenyl),
R¹⁵ is phenyl, or lower alkylphenyl (more preferably C1-C4 alkylphenyl),
A⁸ is lower alkylene (more preferably C1-C4 alkylene, most preferably methylene).

Particularly preferred nonprostanoid prostaglandin I₂ agonists to be used in the practice of the present invention are [3-[[(1S)-2-(4,5-diphenyloxazol-2-yl)-2-cyclohexen-1-yl]methyl]phenoxy]acetic acid, [3-[[(1S)-2-(4,5-diphenyoxazol-2-yl)-2-cyclopenten-1-yl]methyl] phenoxy]acetic acid,
[[(2R)-5-(carboxymethoxy)-2-hydroxy-1,2,3,4-tetrahydronaphth-2-yl]methyl] N,N-diphenylcarbamate,
(1R)-1-[(2R)-2-(4,5-diphenyloxazol-2-yl)pyrrolidin-1-yl]-5-carboxymethoxy-1,2,3,4-tetrahydronaphthalene, [3-[[(2R)-2-(4,5-diphenyloxazol-2-yl)pyrrolidin-1-yl]methyl]phenoxy]acetic acid and salts of these.

The compounds of general formulas (I), (II) and (III) as well as the specific compounds mentioned above are novel or known compounds and can be prepared by the methods described in the publications cited below or methods analogous thereto (those publications cited herein are incorporated by reference):
International publication number: WO 95/17393;
International publication number: WO 95/24393;
International publication number: WO 97/03973.

As other preferred examples of the nonprostanoid prostaglandin I₂ agonist which are to be used in the practice of the present invention, there may be mentioned the following:
(1) Condensed benzene-oxyacetic acid derivatives described in European publication Nos. EP 578847 A, EP 548949 A, EP 542203 Al, EP 581187 A or EP 558062 A (those publications cited herein are incorporated by reference), preferably the compound of the following formula (IV) or salts thereof: 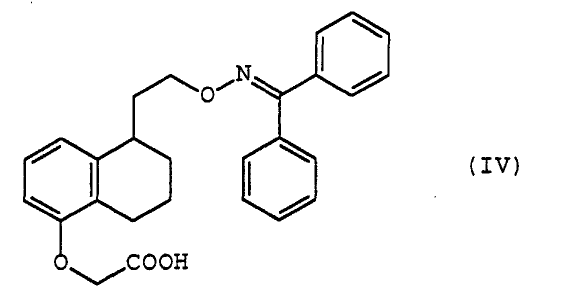
(2) Phenoxyacetic acid derivatives described in U.S. Patent 348969 (this publication cited herein is incorporated by reference), preferably the compound of the following formula (V) or salts thereof: 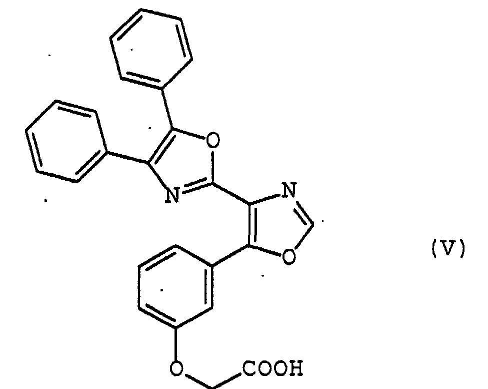
(3) Tricyclic compounds described in International laid-open number WO 98/13356 (this publication cited herein is incorporated by reference), preferably the compound of the following formula (VI) or salts thereof: 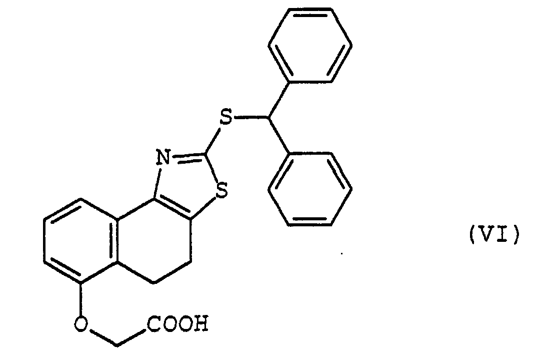 and the like.

The pharmaceutical composition for use in the prevention and/or treatment of skin ulcer according to the present invention can be used in the form of pharmaceutical preparations containing a nonprostanoid prostaglandin I₂ agonist as an active ingredient, for example solids, semisolids or liquids (e.g. tablets, pellets, troches, capsules, suppositories, creams, ointments, aerosols, powders, solutions, emulsions, suspensions, etc.) suited for rectal, pulmonary (nasal or oral inhalation), transnasal, intraocular, external (local), oral or parenteral (including hypodermic, intravenous and intramuscular) routes of administration or for inhalation.

The pharmaceutical composition of this invention may contain one or more of various organic or inorganic carrier substances commonly used for pharmaceutical purposes, for example excipients (e.g. sucrose, starch, mannitol, sorbitol, lactose, glucose, cellulose, talc, calcium phosphate, calcium carbonate, etc.), binding agents (e.g. cellulose, methylcellulose, hydroxypropylcellulose, polypropylpyrrolidone, gelatin, gum arabic, polyethylene glycol, sucrose, starch, etc.), disintegrants (e.g. starch, carboxymethylcellulose, carboxymethylcellulose calcium, hydroxypropylstarch, sodium starchglycolate, sodium hydrogen carbonate, calcium phosphate, calcium citrate, etc.), lubricants (e.g. magnesium stearate, talc, sodium lauryl sulfate, etc.), flavorings or corrigents (e.g. citric acid, menthol, glycine, orange peel powder, etc.), preservatives (e.g. sodium benzoate, sodium hydrogen sulfite, methylparaben, propylparaben, etc.), stabilizers (e.g. citric acid, sodium citrate, acetic acid, etc.), suspending agents (e.g. methylcellulose, polyvinylpyrrolidone, aluminum stearate, etc.), dispersing agents, aqueous diluents (e.g. water) and base waxes (e.g. cacao butter, polyethylene glycol, white petrolatum, etc.).

Generally, the active ingredient may be administered in a unit dose of 0.01 mg/kg to 50 mg/kg once to four times a day. However, the dosage may be increased or decreased according to the age, body weight and condition of the patient or the route of administration.

In cases where it is administered in the form of a lotion, gel or cream, the active substance may be locally administered at a concentration of 0.0001 to 10% (preferably 0.01 to 5%) several times daily, for example twice to five times daily.

### BEST MODES FOR CARRYING OUT THE INVENTION

The test compound used as a representative example of the nonprostanoid prostaglandin I₂ agonist in the practice of the present invention, namely
[[(2R)-5-(carboxymethoxy)-2-hydroxy-1,2,3,4-tetrahydro-2-naphthyl]methyl] N,N-diphenylcarbamate, can be synthesized by the method described in International publication number WO 95/24393 or a method analogous thereto.

### EFFECT OF THE INVENTION

To demonstrate the usefulness of the nonprostanoid prostaglandin I₂ agonist used in the invention for the prevention or treatment of skin ulcer in human or animals, pharmacology test data on a representative compound, namely (1)
[[(2R)-5-(carboxymethoxy)-2-hydroxy-1,2,3,4-tetrahydronaphth-2-yl]methyl] N,N-diphenylcarbamate (hereinafter referred to as test compound), are presented below. Test Example 1 (db/db mouse full-thickness wound model)

### [Experimental]

Using 10-week-old male C57BL/KsJ db/db mice (db/db mice) and the corresponding control C57BL/KsJ +m/+m mice (+m/+m mice), the back hair coat was removed with a depilatory cream. After 3 days, using ophthalmological scissors, the skin in a circular area with a diameter of about 1.5 cm at the dorsal midline was removed to construct a full-thickness wound. Thereafter, the full-thickness wound area was covered with a polyurethane film agent. During 18 consecutive days beginning the day of wound construction, 100 µl of the investigational compound solution or distilled water (vehicle) was taken in a syringe and applied through the polyurethane film once daily. The polyurethane film agent was changed every day and after disinfection with 3% H₂O₂, the drug was applied in the same manner as above. The wound area was measured by the tracing method under light anesthesia every other day as a rule. With the initial wound area at the beginning of drug application being taken as 100%, the fractional area at each day of measurement was calculated.

### [Results]

The wound area in db/db mice expanded slightly in an early stage following construction of the wound and thereafter diminished day by day. Compared with the wound area in +m/+m mice, the area was significantly larger at every measurement day, indicating a delay in healing. In contrast, in the groups treated with 0.01% and 0.1% solutions of the investigational drug, the early expansion of wound area was not observed and the wound area diminished day by day and a significant difference (p<0.05) from the vehicle control group was observed in wound area on and after day 7 and day 4, respectively, following construction of the wound. Comparison in the wound area on the last observation day (day 18 following wound construction) showed that whereas the area reduction in the vehicle treatment group was 73%, the reductions in the groups treated with 0.01% and 0.1% solutions of the investigational compound were 90% and 93%, respectively. These results indicate that the investigational compound has a wound healing-promoting action.

### INDUSTRIAL APPLICABILITY

The above-described nonprostanoid prostaglandin I₂ agonist of the invention is of value in the prevention and/or treatment of skin ulcers (for example, diabetic skin ulcer inclusive of ulcer of lower limb, burn ulcer (burn), traumatic ulcer, crural (cnemial) ulcer, diabetic gangrene, etc.) and decubitus ulcer (bedsore), particularly diabetic skin ulcer, in humans or animals.

## Claims

1. A pharmaceutical composition for the prevention and/or treatment of skin ulcer or bedsore in humans or animals which comprises a nonprostanoid prostaglandin I₂ agonist as an active ingredient.

2. A pharmaceutical composition for the prevention and/or treatment of diabetic skin ulcer in humans or animals which comprises a nonprostanoid prostaglandin I₂ agonist as an active ingredient.

3. A pharmaceutical composition as claimed in Claim 1 or 2, wherein the nonprostanoid prostaglandin I₂ agonist is a compound of the following general formula (I) or a pharmaceutically acceptable salt thereof: 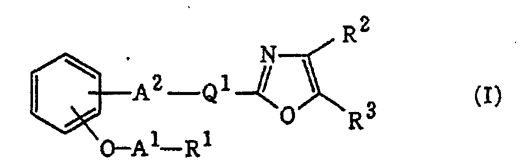 [wherein
R¹ is carboxy or protected carboxy,
R² is aryl which may optionally have one or more suitable substituents,
R³ is aryl which may optionally have one or more suitable substituents,
A¹ is lower alkylene,
A² is a single bond or lower alkylene and
-Q¹- is 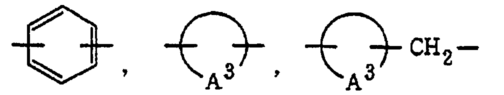
or 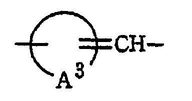 (in which 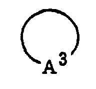 represents cyclo(lower)alkane or cyclo(lower)alkene, which respectively may optionally have one or more suitable substituents)].

4. A pharmaceutical composition as claimed in Claim 1 or 2, wherein the nonprostanoid prostaglandin I₂ agonist is a compound of the following general formula (II) or a pharmaceutically acceptable salt thereof 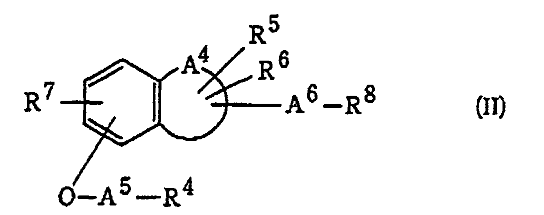 [wherein
R⁴ is carboxy or protected carboxy,
R⁵ is hydrogen, hydroxy or protected hydroxy,
R⁶ is hydrogen, hydroxy, protected hydroxy, lower alkyl or halogen,
R⁷ is hydrogen or halogen,
A⁵ is lower alkylene,
A⁶ is a single bond or lower alkylene and
-R⁸ is 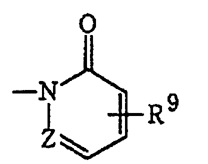
(in which R⁹ is mono(or di or tri)aryl(lower)alkyl and Z is N or CH) or 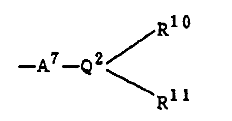 (in which -A⁷- is 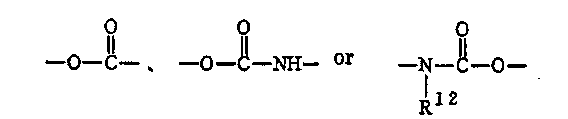 (in which R¹² is hydrogen or lower alkyl), Q² is N or CH, R¹⁰ is aryl and R¹¹ is aryl), and

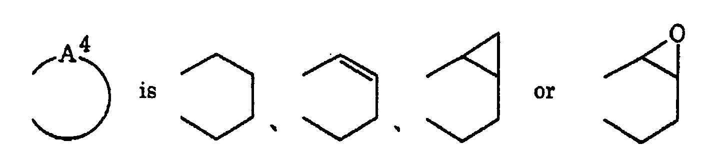

5. A pharmaceutical composition as claimed in Claim 1 or 2, wherein the nonprostanoid prostaglandin I₂ agonist is a compound of the following general formula (III) or a pharmaceutically acceptable salt thereof: 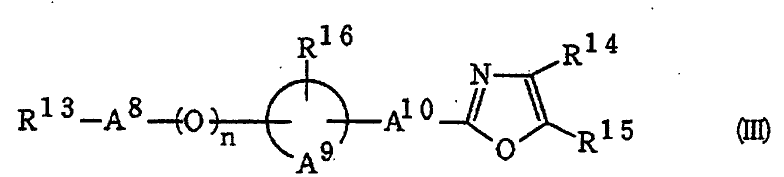 [wherein
R¹³ is carboxy or protected carboxy,
R¹⁴ is aryl which may optionally have one or more suitable substituents,
R¹⁵ is aryl which may optionally have one or more suitable substituents,
R¹⁶ is hydrogen, lower alkyl, hydroxy or aryl,
A⁸ is lower alkylene, 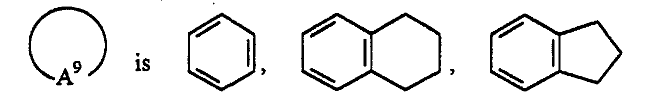 or 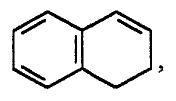
-A¹⁰-is 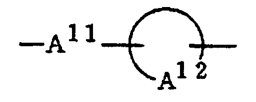
(in which -A¹¹- is a single bond, -CH₂- or -CO-, 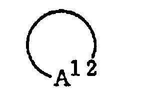 represents cyclo(C5-C8)alkene, cyclo(C7-C8)alkane, bicycloheptane, bicycloheptene, tetrahydrofuran, tetrahydrothiophene, azetidine, pyrrolidine or piperidine, which respectively may optionally have one or more suitable substituents) or
-X-A¹³- (in which -X- is -O-, -S-, or -N(R¹⁷)- (R¹⁷ being hydrogen, lower alkyl or acyl) and A¹³ is lower alkylene which may optionally have one or more suitable substituents) and n is 0 or 1].

6. A pharmaceutical composition as claimed in Claim 1 or 2, wherein the nonprostanoid prostaglandin I₂ agonist is
(1) [3-[[(1S)-2-(4,5-diphenyloxazol-2-yl)-2-cyclohexen-1-yl]methyl]phenoxy]acetic acid,
(2) [3-[[(1S)-2-(4,5-diphenyloxazol-2-yl)-2-cyclopenten-1-yl]methyl]phenoxy]acetic acid,
(3) [(2R)-5-(carboxymethoxy)-2-hydroxy-1,2,3,4-tetrahydronaphth-2-yl]methyl] N,N-diphenylcarbamate,
(4) (1R)-1-[(2R)-2-(4,5-diphenyloxazol-2-yl)pyrrolidin-1-yl]-5-carboxymethoxy-1,2,3,4-tetrahydronaphthalene or
(5) [3-[[(2R)-2-(4,5-diphenyloxazol-2-yl)pyrrolidin-1-yl]methyl]phenoxy]acetic acid, or a pharmaceutically acceptable salt thereof.

7. The use of a nonprostanoid prostaglandin I₂ agonist in the manufacture of pharmaceutical compositions for use in the prevention and/or treatment of skin ulcer or bedsore in humans or animals.

8. A method for the prevention and/or treatment of skin ulcer or bedsore which comprises administering an effective amount of a nonprostanoid prostaglandin I₂ agonist to a human or animal requiring such prevention and/or treatment.
